# EUROPÄISCHE PATENTSCHRIFT

(11) **EP 2 462 904 B1**
(45) Veröffentlichungstag und Bekanntmachung des Hinweises auf die Patenterteilung: **01.06.2016**
(21) Anmeldenummer: 11009819.1
(22) Anmeldetag: 13.12.2011
(51) Int. Cl.: A61F 5/01

(54) **Orthese**
Orthotic
Orthèse

(30) Priorität: 13.12.2010 DE 102010054282
(43) Veröffentlichungstag der Anmeldung: 13.06.2012
(73) Patentinhaber: Pohlig GmbH, 83278 Traunstein (DE)
(72) Erfinder: Pohlig-Wetzelsperger, Claudia, 83278 Traunstein (DE)
(74) Vertreter: Paustian, Othmar

(56) Entgegenhaltungen:
- DE-A1- 19 801 951
- US-A- 5 219 323
- US-A- 5 662 594
- US-A1- 2005 165 337
- US-A1- 2009 030 353
- US-A1- 2009 054 820
- US-A1- 2010 204 804

## Beschreibung

Die Erfindung betrifft eine Orthese zur Anlage an einen Unterarm, gemäß dem Oberbegriff von Anspruch 1.

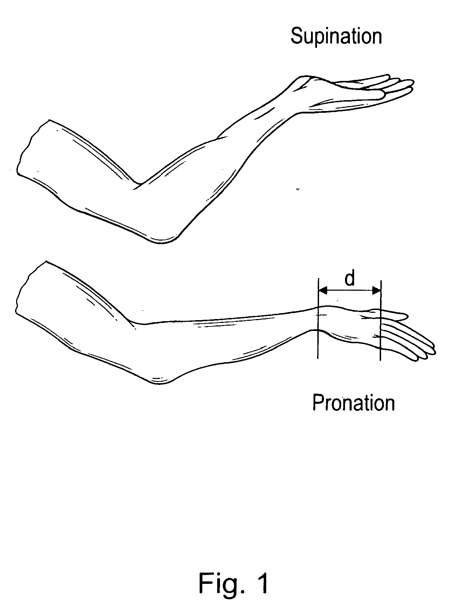

Der menschliche Unterarm kann um seine Längsrichtung gedreht werden. Eine Drehung bei der die Handfläche nach oben gewendet wird heißt Supination und eine Drehung bei der die Handfläche nach unten gewendet wird heißt Pronation (vgl. Figur 1). Supination und Pronation werden jeweils als Umwendebewegung bezeichnet.

Speiche (Radius) und Elle (Ulna) sind gelenkig durch das proximale, körpernahe, Radioulnargelenk (PRU) und das distale, körperferne, Radioulnargelenk (DRU) verbunden. "Proximal" ist hier auch als "oberarmnah" zu verstehen und "distal" als "oberarmfern". Das PRU ist dem Ellenbogengelenk zugeordnet und das DRU ist dem Handgelenk zugeordnet. Das DRU, oder auch körperfernes bzw. unteres Ellen-Speichen-Gelenk, wird mit den distalen Enden der Elle (Ellenkopf, Caput ulnae) und der Speiche (Epiphysis distalis radii) gebildet. Es dreht sich unter Beteiligung des körpernahen Radioulnargelenks und ermöglicht so die Umwendebewegungen des Unterarms.

Bei einer Umwendebewegung drehen sich Elle und Speiche um die biomechanische Drehachse. Damit sich PRU und DRU nicht gegenseitig blockieren und eine Umwendebewegung möglich ist, sind die Achsen der Radioulnargelenke im wesentlichen koaxial zueinander ausgerichtet. Vereinfacht kann die biomechanische Drehachse als eine Linie gedacht werden, die zwischen Elle und Speiche jeweils mittig durch die beiden Radioulnargelenke verläuft. Elle und Speiche drehen sich bei einer Umwendebewegung um diese gedachte Linie.

Funktional werden zwei Muskelgruppen unterschieden, die entweder zur Pronations- oder zur Supinationsbewegung beitragen. Der Musculus supinator setzt am proximalen Ende des Radius an und fungiert als Abroller, während der Bizepsmuskel, auch als Hauptsupinator bezeichnet, die radiale Tuberositas festschnallt- Der Musculus brachioradialis fungiert nur bei vollständiger Unterarmpronation als Supinator. Der relativ schwache Musculus pronator teres setzt im Apex der Pronatorbeuge des Radius an, während der Musculus pronator quadratus sich um das distale Ulnaende windet und als Abrollen fungiert.

Weitere Stabilisatoren betreffend die Funktion von Radius und Ulna sind neben den Ligamenta der beiden Radioulnargelenke vor allem die Membrana interossea, sowie die Muskulatur des Unterarms.

Zahlreiche Indikationen erfordern die Einstellung bzw. vorübergehende Festlegung des Unterarms. Konventionell wird dazu der Unterarm, gegebenenfalls einschließlich des Ellenbogengelenks und/oder des Handgelenks, mit einem Gips oder einem Kunststoffverband fixiert.

Dabei droht insbesondere eine irreversible Einschränkung oder gar eine Blockade der Umwendebewegung oder auch eine Atrophie der Muskulatur. Besonders schädlich ist ein Zusammenwachsen der Knochenhaut zwischen der Elle und der Speiche.

Etwa nach Sehnenverpflanzungen gehören physiotherapeutische Übungen zur Kräftigung der Muskulatur und zur Erhaltung der Pronationsbewegung und der Supinationsbewegung zum Standard; dazu muss der Verband jedoch abgenommen werden.

Orthesen sind grundsätzlich bekannt. Sie werden seit Jahren insbesondere zur Stützung von Gelenken und von Extremitäten insgesamt eingesetzt.

Aus der US-A-2009/054820 ist eine Orthese mit einem distalen Schalenteil bekannt, das den Unterarm und/oder die Hand in einem Hand/Handgelenk-Bereich drehfest fasst, und mit einem proximalen Schalenteil, das den Ellenbogen relativ zum Oberarm drehfest fasst. Das distale Schalenteil ist mit einer Positionier-Baugruppe verbunden und das proximale Schalenteil mit einer Befestigungsvorrichtung. Die Positionier-Baugruppe und die Befestigungsvorrichtung wiederum sind durch ein Verlängerungselement miteinander verbunden, wobei diese Verbindung biegesteif ausgeführt ist. Die Positionier-Baugruppe weist ein Ringelement auf, das mit dem distalen Schalenteil verbunden ist und gegenüber der restlichen Positionier-Baugruppe und damit gegenüber dem Verlängerungselement sowie der Befestigungsvorrichtung drehbar ist, sodass mittels des Ringelementes das distale Schalenteil relativ zum proximalen Schalenteil zur Durchführung der Umwendebewegung kraftfrei drehbar ist.

Bei der aus der gattungsgemäßen US 5 662 594 bekannten Orthese können die gelenkig miteinander verbundenen Schalenteile zueinander vollständig frei beweglich sein oder in einem begrenzten Bereich zueinander frei beweglich sein oder relativ zueinander fixiert werden. Die Fixierung erfolgt mittels Schrauben, die im Überlappungsbereich zweier Schalenteile runde Durchgangslöcher in dem außenliegenden Schalenteil durchlaufen und in dem innenliegenden Schalenteil verschraubt sind. Eine freie Bewegung nur innerhalb eines begrenzten Bereiches wird erreicht, indem die Befestigungsschrauben in dem außenliegenden Schalenteif ein Langloch durchlaufen, dessen Längserstreckung eine freie Bewegung ermöglicht und begrenzt, und in dem innenliegenden Schalenteil verschraubt sind. Auf diese Weise ist es möglich, das distale Unterarm-Schalenteil gegenüber dem proximalen Unterarm-Schalenteil längs der Längserstreckung des Langloches zu verschwenken und somit eine entsprechende Umwendebewegung des Unterarms durchzuführen,

Demgegenüber liegt die Aufgabe der vorliegenden Erfindung darin, eine gattungsgemäße Orthese so zu verbessern, dass ein Beüben von Umwendebewegungen mit gezielter Stärkung der Muskelkraft möglich ist.

Die Erfindung basiert dabei auf der Idee, eine Orthese anzugeben, die selbst beim Tragen derselben das Beüben von Umwendebewegungen erlaubt.

Die Aufgabe wird bei einer Orthese der eingangs genannten Art gelöst durch ein an einem der beiden Schalenteile befestigtes Gegenicraftelement, das während der Durchführung einer Umwendebewegung des Unterarms eine der Umwendebewegung entgegenwirkende Gegenkraft ausübt.

Bevorzugte Ausgestaltungen der Erfindung finden sich in den abhängigen Ansprüchen und in der nachfolgenden Beschreibung.

Bei einer Umwendebewegung dreht sich immer auch das untere Ellen-Speichen-Gelenk, also das distale Radioulnargelenk.

Das distale Schalenteil greift dieses untere Ellen-Speichen-Gelenk bzw. das Handgelenk und/oder die Hand so, dass es sich bei einer Umwendebewegung zusammen mit dem unteren Ellen-Speichen-Gelenk und der Hand gleichsinnig dreht. Dazu kann es das Handgelenk bzw. die Hand innerhalb eines ganzen Bereichs, hier als Hand/Handgelenk-Bereich bezeichnet, an einer Position oder an mehreren Positionen fassen.

Der Hand/Handgelenk-Bereich beginnt mit dem unteren Ellen-Speichen-Gelenk und erstreckt sich weiter über das Handgelenk, den proximalen Teil der Mittelhand, bis hin zum distalen Teil der Mittelhand. Der Hand/Handgelenk-Bereich endet mit dem distalen Teil der Mittelhand, also unmittelbar nach dem distalen Ende der Mittelhandknochen.

Das proximale Schalenteil fasst den Ellenbogen so, dass es relativ zum Oberarm drehfest ist. Das heißt, wenn der Unterarm eine Umwendebewegung ausführt, bleibt das proximale Schalenteil relativ zum Oberarm ortsfest.

Proximales und distales Schalenteil sind also beide mit Blick auf einen Teil des Arms drehfest. Das proximale Schalenteil ist drehfest zum Oberarm und das distale Schalenteil folgt bei einer Drehung der Drehung des unteren Ellen-Speichen-Gelenks; ist also relativ zu diesem drehfest. Es kann jedoch für beide Schalenteile jeweils ein gewisses Spiel gegeben sein, beispielsweise bis zu 20°, besser nur bis zu 10°, oder auch nur bis zu 5° bzw. 2°.

Es ist bevorzugt die Schalenteile jeweils zumindest abschnittweise formangepasst auszugestalten. Das erleichtert einen hinreichenden Halt und verringert das Spiel bei der Umwendebewegung.

Die Schalenteile können als Vollschalen oder auch als Teilschalen ausgebildet sein. Sind sie als Teilschalen ausgebildet, bietet es sich an, diese mit Gurten an dem Unterarm bzw. der Hand zu befestigen.

Die beiden Schalenteile drehen relativ zueinander bzw. das distale Schalenteil dreht relativ zum proximalen Schalenteil um die biomechanische Achse des Unterarms. Dazu sind sie über ein Drehgelenk miteinander verbunden, dessen Drehachse mit der biomechanischen Achse übereinstimmt.

Das die beiden Schalenteile drehbar miteinander verbindende Drehgelenk ist vorzugsweise im mittleren Drittel der Orthese, gemessen vom Handgelenk bis zum Ellenbogen, und besonders bevorzugt mittig zwischen Ellenbogengelenk und Handgelenk angeordnet.

Das distale Schalenteil und das proximale Schalenteil bilden zwischen Ellenbogengelenk und Handgelenk keine drehfeste Verbindung mit dem Unterarm aus. Die beiden Schalenteile und das Drehgelenk können z. B. in der Umgebung des Drehgelenks von dem Unterarm abgehoben bzw. von diesem beabstandet sein, um bei der Drehung keine unnötige Reibung an dem Unterarm zu erzeugen bzw. um überhaupt eine leichte Drehung der beiden Schalenteile relativ zueinander zu erlauben. Vorzugsweise sind die Schalenteile in Längsrichtung beidseits des Drehgelenks jeweils über eine Lange von zumindest 3 cm, 5 cm oder auch 7 cm und das Drehgelenk selbst von dem Unterarm abgehoben.

Das Drehgelenk ist so ausgelegt, dass es nur eine Drehung der beiden Schalenteile relativ zueinander um die biomechanische Achse des Unterarms freigibt. Dabei ist es so ausgelegt, dass die beiden Schalenteile relativ zueinander in der angegebenen Reihenfolge zunehmend bevorzugt um 60°, 120°, 180° und 210° relativ zueinander drehbar sind.

Die Orthese ist dabei insgesamt auch biegesteif ausgeführt, so dass keine anderen Bewegungen als eine Drehung zur Ausführung einer Umwendebewegung möglich sind. Ungewollte Bewegungen und ein Hineindrehen in Fehlstellungen können so vermieden werden. Zusätzlich können die beiden Schalenteile schaftartig ausgelegt sein.

Weiter kann die Orthese über eine Arretierung vertügen, mit der die Drehung der beiden Schalenteile relativ zueinander lösbar unterbunden werden kann. Dies ermöglicht ein Beüben der Umwendebewegung zu bestimmten Zeiten und eine ansonsten feste Einstellung des Drehwinkels, etwa in Pronation oder Supination.

Der Winkel im Ellenbogen und die Lage des Handgelenks können bei erfindungsgemäßen Orthesen frei gewählt werden. Je nach Ausgestaltung der Schalenteile kann das Handgelenk und/oder das Ellenbogengelenk auch in einer gewünschten Lage fixiert werden.

Die beiden Schalenteile brauchen zum drehfesten Fassen nicht unmittelbar an dem Unterarm bzw. der Hand anzuliegen; es kann sich beispielsweise ein Textil, ein Polster, ein sogenanntes "Lining" oder auch Kleidung zwischen dem Unterarm bzw. der Hand und den Schalenteilen befinden.

Der Träger der Orthese kann die Umwendebewegungen beispielsweise aus eigener Kraft durchführen, oder aber die beiden Schalenteile werden von außen, etwa durch einen Physiotherapeuten, relativ zueinander bewegt.

Die erfindungsgemäße Orthese erlaubt einerseits, einen Unterarm einschließlich Ellenbogengelenk und Handgelenk wie mit einem herkömmlichen Gips oder Kunststoffverband zu fixieren, und unterstützt andererseits ein einfaches Beüben von Pronation und Supination. Das Beüben der Umwendebewegungen wirkt sich positiv auf die Muskulatur und die Gelenke bzw. das Skelett aus, so dass die Degenerationsphänomene, wie sie bei dauerhaft getragenen konventionellen Verbänden auftreten können, vermieden werden.

Besonders bevorzugt ist es, mit dem distalen Schalenteil die Mittelhand zu fassen. Das distale Schalenteil kann hier besonders gut angepasst werden - und eine Umwendebewegung des Unterarms und eine Drehung der Hand gehören in der Regel zwingend zusammen; Ausnahmen sind etwa für den Fall durchtrennter Bänder möglich. Bei dieser Ausführungsform erstreckt sich das distale Schalenteil über das Handgelenk hinweg, braucht dieses aber nicht zu fassen oder zu berühren.

Vorzugsweise weist das Drehgelenk in einer Ebene senkrecht zur Drehachse einen inneren kreisbogenförmigen Gelenkabschnitt und einen darüber liegenden äußeren kreisbogenförmigen Gelenkabschnitt auf, die zur Durchführung der Umwendebewegung aneinander vorbeiführbar sind.

Die Kreisbögen sind vorzugsweise flächig ausgelegt, wobei die Flächennormale jeweils vom Unterarm wegweist.

Die beiden kreisbogenförmigen Gelenkabschnitte können zur Ausführung der Drehung aneinander vorbeigleiten. Dazu können sie etwa über ein Gleitlager verfügen. Es ist bevorzugt, ein Kugellager zwischen den beiden kreisbogenförmigen Gelenkabschnitten vorzusehen. Grundsätzlich ist auch ein Walzlager denkbar. Beides verringert die Reibung; Kugellager sind ökonomisch besonders vorteilhaft.

Der äußere kreisbogenförmige Gelenkabschnitt kann als Schale ausgebildet sein und der darunter liegende innere kreisbogenförmige Gelenkabschnitt als gekrümmte Platte. Die Schale kann die Platte proximal und distal umgreifen und so die Platte durch die Schale geführt werden. Grundsätzlich kann auch einer der beiden kreisbogenförmigen Gelenkabschnitte als Schiene ausgebildet sein und der andere der beiden kreisbogenförmigen Gelenkabschnitte diese Schiene umfassen.

Man spricht in diesem Zusammenhang auch von einem Schienengelenk bzw. Schlittengelenk oder auch Halbschalengelenk,

Kreisbogenförmige Gelenkabschnitte sind bevorzugt; grundsätzlich sind jedoch auch Vollkreise denkbar.

Bei einer bevorzugten Ausführungsform der Erfindung verfügt die Orthese über einen Motor zum Antrieb der Drehung des distalen Schalenteils relativ zum proximalen Schalenteil.

Bei dem Motor handelt es sich vorzugsweise um einen Elektromotor. Der Motor kann die beiden Schalenteile etwa so relativ zueinander drehen, dass sich abwechselnd eine Pronation und eine Supination ergibt. Der Motor kann beispielsweise den Orthesenträger bei der Umwendebewegung unterstützen. Es ist auch denkbar, dass der Motor, etwa gegen den Widerstand von Muskeln und Bändern, eine bestimmte Einstellung, also einen bestimmten Drehwinkel, zwischen den beiden Schalenteilen, erzwingt.

Über eine Motorsteuerung können gewünschte Winkelintervalle und Unterstützungen zusammen mit dem gewünschten zeitlichen Ablauf eingestellt werden, so dass die Muskeln bzw. Muskelgruppen genau definiert beübt werden können.

Erfindungsgemäß verfügt die Orthese über ein Gegenkraftelement zur Ausübung einer eine (durch Muskelkraft bewirkte) Umwendebewegung belastenden Gegenkraft, d. h. zur Ausübung einer einer Muskelkraft entgegenwirkenden Gegenkraft.

Versucht der Orthesenträger eine Umwendebewegung auszuführen, so wirkt das Gegenkraftelement dagegen. So können Umwendebewegungen besonders intensiv geübt werden.

Bei dem Gegenkraftelement kann es sich dabei beispielsweise um den oben erwähnten Motor handeln. Dieser kann dann gegen die vom Orthesenträger aufgewendete Kraft arbeiten.

Vorzugsweise handelt es sich bei dem Gegenkraftelement um ein Federelement. Dabei kann es sich etwa um eine Metallfeder, einen Gummizug oder um eine Gasdruckfeder handeln, Es ist besonders bevorzugt als Gegenkraftelement eine Gasdruckfeder einzusetzen, da eine Gasdruckfeder nahezu unabhängig von dem Federweg die gleiche Kraft ausübt.

Das Federelement ist fix an einem der beiden Schalenteile befestigt und dabei vorzugsweise über einen Seilzug mit dem anderen der beiden Schalenteile verbunden.

Eine Umwendebewegung, etwa aus einer voreingestellten Ausgangsstellung heraus, kann so eine Belastung des Federelements bewirken, wobei sich das Federelement bei Rückführung in die Ausgangsstellung wieder entlastet; wird der Seilzug anders verlegt, kann das Federelement bei einer Umwendebewegung aus einer Ausgangsstellung heraus auch entlastet werden und bei der Rückführung in die Ausgangsstellung wieder belastet werden.

Bei einem vorrangig linearen Federelement ist es bevorzugt, dieses parallel zur Drehachse des Drehgelenks auszurichten; also parallel zur biomechanischen Drehachse.

Gemäß einigen Indikationen ist es wünschenswert, den Unterarm mit einem bestimmten Winkel, etwa in Supination oder Pronation, voreinzustellen. Daher ist es bevorzugt, dass bei der Orthese die beiden Schalenteile relativ zueinander um die Drehachse des Drehgelenks gegenkraftfrei in eine Ausgangsstellung drehbar sind, aus der heraus das distale Schalenteil relativ zum proximalen Schalenteil zur Durchführung der Umwendebewegung entgegen der Gegenkraft drehbar ist.

Die Voreinstellung des Unterarms mit einem bestimmten Drehwinkel ist auf diese Weise durch gegenkraftfreies Drehen der beiden Schalenteile relativ zueinander um die Drehachse des Drehgelenks um den einzustellenden Winkel möglich.

Vorzugsweise ist dabei das distale Schalenteil aus der Ausgangsstellung heraus in beide Umwenderichtungen drehbar und das Gegenkraftelement dazu ausgelegt, in beiden Umwenderichtungen eine der Drehung entgegenwirkende Gegenkraft auszuüben. Dazu kann sie gegebenenfalls zwischen einer Gegenkraftbelastung für Supination und einer für Pronation umgeschaltet werden. Das Umschalten kann beispielsweise durch das Umlegen eines Seilzugs geschehen. Gegebenenfalls können dazu auch zwei Seilzüge vorgesehen werden.

Jede Drehung aus der Ausgangsstellung der Orthese heraus kann daher mit einer Gegenkraft belastet sein, so dass die Orthese den Unterarm des Orthesenträgers wieder in die Ausgangsstellung zurückdreht, wenn dieser nicht genug Kraft gegen die Gegenkraft aufwendet. Gegebenenfalls wird auch in der Ausgangsstellung eine gewisse Kraft gegen die Bänder und/oder die Muskulatur des Orthesenträgers ausgeübt; dies kann etwa gewünscht sein, um nichtknöcherne Strukturen zu dehnen.

In diesem Zusammenhang, also bei einer Orthese mit Gegenkraftelement, kann die eingestellte Ausgangsstellung auch als ein Sollzustand der Orthese betrachtet werden, von dem aus der Orthesenträger nur gegen den Widerstand des Gegenkraftelements abweichen kann. Ist die Gegenkraft größer als die vom Orthesenträger aufgewendete umwendende Kraft, so kehrt die Orthese in den eingestellten Zustand zurück.

Weiter ist es bevorzugt, das proximale Schalenteil mit einem Streckanschlag für das Ellenbogengelenk auszustatten, so dass der Ellenbogen in einem gewissen Winkel, z. B. 15°, gestützt wird und das proximale Schalenteil besonders drehfest angelegt werden kann.

Insbesondere kann das proximale Schalenteil ellenbogenübergreifend ausgelegt sein und ggf. auch eine Gelenkschiene umfassen.

Die in der vorangehenden und in der folgenden Beschreibung offenbarten Aspekte können auch in anderen als den gezeigten Kombinationen erfindungswesentlich sein.

Im folgenden soll die Erfindung auch anhand von Ausführungsbeispielen näher erläutert werden, ohne dabei die Erfindung durch die Beispiele einschränken zu wollen:
Figur 1 zeigt schematisch einen Unterarm in Supination und einen Unterarm in Pronation;
Figur 2 zeigt die einem Unterarm abgewandte Außenseite einer erfindungsgemäßen Orthese;
Figur 3 zeigt die Innenseite der Orthese aus Figur 2; und
Figur 4 zeigt ein Drehgelenk für eine erfindungsgemäße Orthese mit einem Seilzug.

Figur 1 zeigt oben einen Unterarm in Supination und unten einen Unterarm in Pronation. Bei dem unten dargestellten Unteram ist ein Bereich "d" markiert, der zum drehfesten Fassen durch das distale Ende einer erfindungsgemäßen Orthese geeignet ist; der hier so genannte Hand/Handgelenk-Bereich.

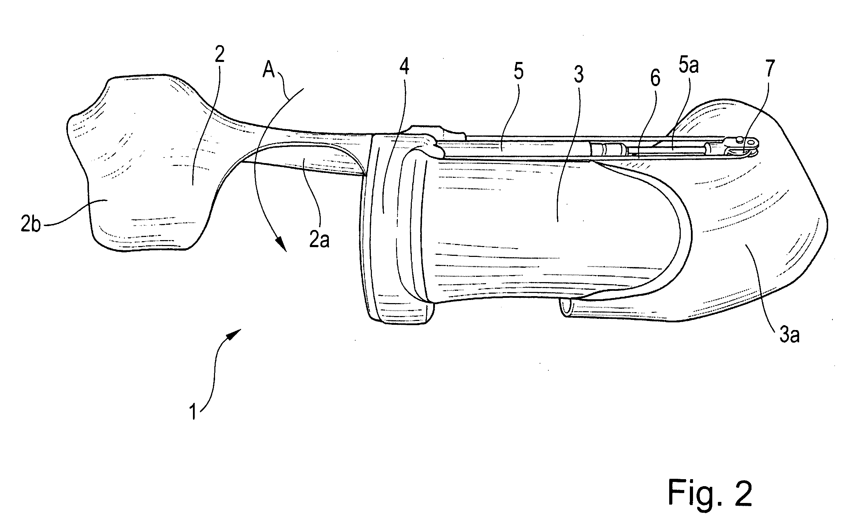

Figur 2 zeigt eine erfindungsgemäße Orthese 1 von ihrer äußeren, unterarmabgewandten, Seite.

Das dargestellte Ausführungsbeispiel einer erfindungsgemäßen Orthese weist ein distales Schalenteil 2 mit einer Brücke 2a, ein proximales Schalenteil 3 mit einem proximalen Teil 3a, ein Drehgelenk 4 und eine Gasdruckfeder 5 mit einem Kolben 5a sowie einen Seilzug 6 mit einer Umlenkrolle 7 auf.

Die Schalenteile 2 und 3 sind aus einem Karbonmaterial gefertigt.

Das distale Schalenteil 2 ist biege- und drehfest ausgebildet. Es erstreckt sich von dem etwa mittig gelegenen Drehgelenk 4 einstückig über eine vergleichsweise schmale Brücke 2a bis hin zu ihrem distal gelegenen Kontakt- bzw. Endabschnitt 2b, der dazu ausgelegt ist, eine Hand formangepasst und flächig, unter Einbeziehung der Mittelhand, drehfest teilweise zu umfassen.

Das proximale Schalenteil 3 ist biege- und drehfest ausgebildet. Es erstreckt sich ausgehend von dem Drehgelenk 4 bis zum proximalen Ende des den Ellenbogen drehfest übergreifenden Teils 3a. Der ellenbogenübergreifende Teil 3a ist als Streckanschlag ausgelegt, der einen Ellenbogenwinkel von mindestens 15° sicherstellen soll.

Das Drehgelenk 4 weist eine Drehachse auf, die im an den Unterarm angelegten Zustand der Orthese mit der biomechanischen Drehachse des Unterarms übereinstimmt. Es ist aus zwei kreisbogenförmigen Gelenkabschnitten gebildet. Die beiden Gelenkabschnitte, ein innerer und ein äußerer, sind senkrecht zur Drehachse ausgerichtet. In Figur 2 blickt man von außen auf die äußere Seite des äußeren Gelenkabschnitts, unter dem der innere Gelenkabschnitt (nicht zu erkennen) liegt.

Der innere Gelenkabschnitt ist drehfest mit dem distalen Schalenteil 2 verbunden und ist als eine kreisbogenförmige Platte aus nichtrostendem Stahl ausgebildet. Der äußere Gelenkabschnitt ist, wie die beiden Schalenteile 2, 3, aus Karbon angefertigt; er fasst über die Seiten der Platte des inneren Gelenkabschnitts. Die Gelenkabschnitte sind gegeneinander 8-fach kugelgelagert.

In anderen Worten: Der äußere Gelenkabschnitt greift proximal und distal über den inneren Gelenkabschnitt längs dessen Längserstreckung. Die Platte des inneren Gelenkabschnitts kann sich in dem äußeren Gelenkabschnitt bewegen, sodass das distale Schalenteil 2 relativ zum proximalen Schalenteil 3 um die Längsrichtung der Orthese 1 bzw. um die biomechanische Drehachse des Unterarms drehen kann. Die mögliche Drehung ist durch den Pfeil A in Figur 2 angedeutet.

Die Gasdruckfeder 5 ist fix mit dem proximalen Schalenteil 3 verbunden. Sie ist axial ausgerichtet, wobei sie mit einem ihrer beiden Enden unmittelbar proximal des Drehgelenks 4 befestigt ist und das andere Ende mit der Kolbenstange 5a in Richtung ellenbogenübergreifenden Teil 3a weist.

Am freien Ende der Kolbenstange 5a ist eine Umlenkrolle 7 vorgesehen. Der Seilzug 6 ist in Figur 2 gerade unterhalb der Gasdruckfeder 5 unmittelbar proximal des Drehgelenks 4 am proximalen Schalenteil 3 befestigt und dann von dort zur Umlenkrolle 7 und über diese wieder zurück in Richtung Drehgelenk 4 geführt. Dort wird der Seilzug 6 über eine weitere Umlenkung (nicht gezeigt) zwischen den beiden kreisbogenförmigen Gelenkabschnitten geführt (nicht gezeigt), um im unteren Teil der Figur 2 an dem inneren kreisbogenförmigen Gelenkabschnitt anzugreifen (nicht gezeigt).

Wird das distale Schalenteil 2 relativ zu dem proximalen Schalenteil 3 in Richtung des Pfeils A gedreht, so wird über den Seilzug 6 und die Umlenkrolle 7 der Kolben 5a in die Gasdruckfeder 5 eingeschoben und die Gasdruckfeder 5 dabei komprimiert. Dabei baut sich eine Gegenkraft auf. Ist die Gegenkraft größer als die das Schalenteil 2 in Richtung A drehende Kraft, so bewirkt die Gasdruckfeder 5 ein Zurückdrehen des Schalenteils 2 in die Ausgangsstellung.

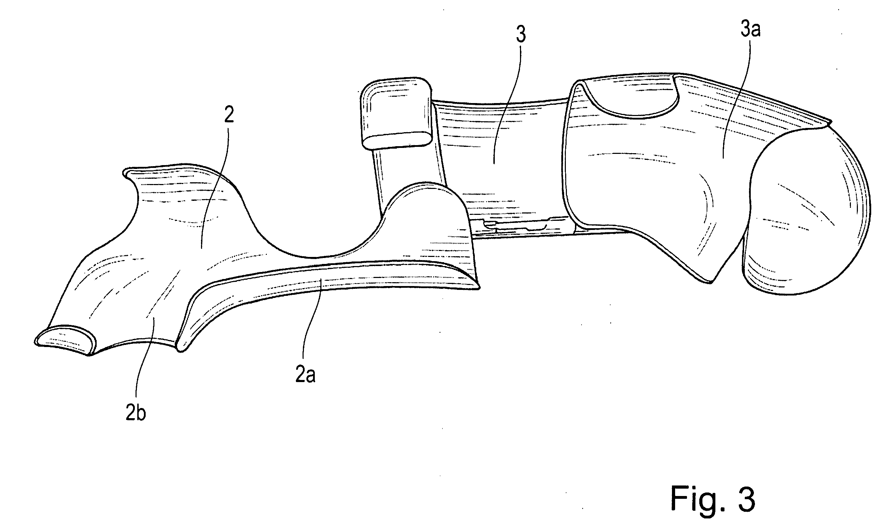

Figur 3 zeigt die Orthese aus Figur 2 von ihrer inneren, bei angelegter Orthese der dem Unterarm zugewandten Seite. Hier ist besonders gut erkennbar, dass die Schalenteile 2 und 3, insbesondere mit ihren Abschnitten 3a und 2b, für eine drehfeste Anlage formangepasst ausgelegt sind. Der Kontaktabschnitt 2b des distalen Schalenteils 2 eignet sich für die Aufnahme der Mittelhand, insbesondere des Daumenballens. Der Abschnitt 3a des distalen Schalenteils 3 ist so ausgelegt, dass das proximale Schalenteil 3 relativ zum Oberarm drehfest an den Ellenbogen angelegt werden kann.

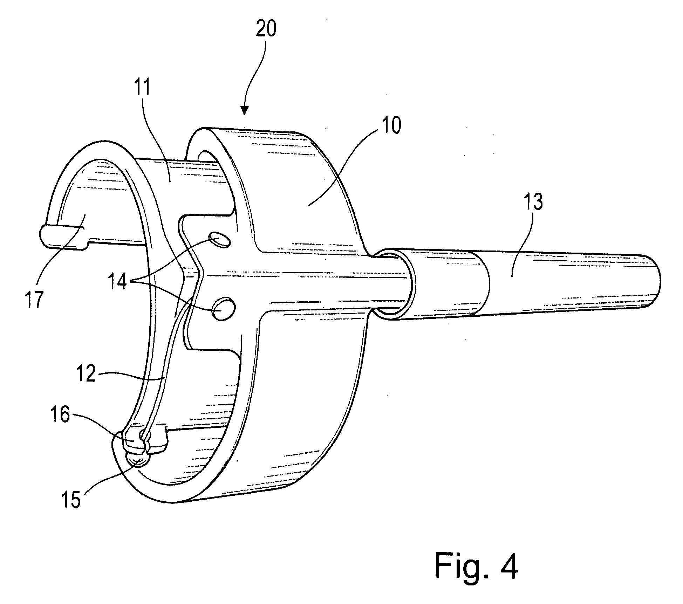

Figur 4 zeigt ein Drehgelenk 20 für eine erfindungsgemäße Orthese, das zwischen einer Gegenkraftbelastung in Supination und einer Gegenkraftbelastung in Pronation umgeschaltet werden kann. Auch dieses Gelenk verfügt über einen äußeren kreisbogenförmigen Gelenkabschnitt 10 und über einen inneren kreisbogenförmigen Gelenkabschnitt 11.

Der innere Gelenkabschnitt 11 ist relativ zum äußeren Gelenkabschnitt 10 drehbar gelagert. Ein Seilzug 12 läuft in einer Aufnahme 16 zu einem Ende des inneren Gelenkabschnitts 11 und wird dort mittels eines Stoppknopfes 15 gehalten, der an dem dortigen Ende des Seilzuges 12 unverschieblich befestigt ist und an einem Anschlag des inneren Gelenkabschnitts 11 anliegt. Das andere Ende des Seilzuges 12 wird über eine Umlenkung 14 einem Federelement in einer Umhüllung 13 zugeführt. Hier ist die Aufnahme 16 als Rille 16 ausgebildet, in die der Seilzug 12 einfach eingelegt werden kann. Am anderen Ende des inneren Gelenkabschnitts 11 befindet sich eine ebensolche Aufnahme 17 mit Anschlag (nicht zu erkennen).

Um dieses Drehgelenk umzuschalten, muss nur der Seilzug 12 aus der Aufnahme 16 entnommen und in die Aufnahme 17 eingelegt werden.

### Bezugszeichenliste

- d: Hand/Handgelenk-Bereich
- A: Drehrichtung
- 1: Orthese
- 2: distales Schalenteil
- 2a: Brücke
- 2b: formangepasster Abschnitt
- 3: proximales Schalenteil
- 3a: ellenbogenübergreifender Teil
- 4: Drehgelenk
- 5: Gasdruckfeder
- 5a: Kolbenstange
- 6: Seilzug
- 7: Umlenkrolle
- 10: äußerer Gelenkabschnitt
- 11: innerer Gelenkabschnitt
- 12: Seilzug
- 13: Umhüllung
- 14: Umlenkung
- 15: Stoppknopf
- 16: Aufnahme
- 17: Aufnahme
- 20: Drehgelenk

## Patentansprüche

1. Orthese (1) zur Führung einer Umwendebewegung eines Unterarms um dessen biomechanische Drehachse, mit
einem distalen Schalenteil (2), dazu ausgelegt, den Unterarm und/oder die Hand in einem Kontaktabschnitt (2b), der in dem sich von dem distalen Ellen-Speichen-Gelenk bis zum distalen Ende der Mittelhand erstreckenden Hand/Handgelenk-Bereich (d) liegt, drehfest zu fassen, so dass das distale Schalenteil (2) und der Hand/Handgelenk-Bereich (d) gemeinsam um die biomechanische Drehachse des Unterarms drehbar sind,
einem proximalen Schalenteil (3), dazu ausgelegt, den Ellenbogen relativ zum Oberarm drehfest zu fassen, und mit
einem Drehgelenk (4, 20) mit einer Drehachse, die im angelegten Zustand der Orthese mit der biomechanischen Drehachse des Unterarms übereinstimmt und um die herum das distale Schalenteil (2) relativ zum proximalen Schalenteil (3) zur Durchführung der Umwendebewegung drehbar ist,
wobei das Drehgelenk (4, 20) die beiden Schalenteile (2, 3) miteinander verbindet und die Verbindung biegesteif ausgeführt ist,
**gekennzeichnet durch**
ein an einem der beiden Schalenteile (2, 3) befestigtes Gegenkraftelement (5), das während der Durchführung einer Umwendebewegung des Unterarms eine der Umwendebewegung entgegenwirkende Gegenkraft ausübt.

2. Orthese (1) nach Anspruch 1, bei der das distale Schalenteil (2), dazu ausgelegt ist, die Mittelhand zu fassen.

3. Orthese (1) nach Anspruch 1 oder 2, bei der das Drehgelenk (4) in einer Ebene senkrecht zur Längsdrehachse einen inneren kreisbogenförmigen Gelenkabschnitt und einen darüber liegenden äußeren kreisbogenförmigen Gelenkabschnitt aufweist, die zur Durchführung der Umwendebewegung aneinander vorbeiführbar sind.

4. Orthese (1) nach einem der Ansprüche 1 bis 3, mit einem Motor zum Antrieb der Drehung des distalen Schalenteils (2).

5. Orthese (1) nach einem der vorangehenden Ansprüche, bei der das Gegenkraftelement (5) ein Federelement (5) ist.

6. Orthese (1) nach Anspruch 5, bei der das Gegenkrafteleiment (5) eine Gasdruckfeder (5) ist.

7. Orthese (1) nach Anspruch 5 oder 6, bei der das Federelement (5) über einen Seilzug (6, 12) mit dem anderen der beiden Schalenteile (2, 3) verbunden ist.

8. Orthese (1) nach Anspruch 7, bei der das Federelement (5) parallel zur Drehachse des Drehgelenks ausgerichtet ist.

9. Orthese (1) nach einem der vorangehenden Ansprüche, bei der die beiden Schalenteile (2, 3) relativ zueinander um die Drehachse des Drehgelenks gegenkraftfrei in eine Ausgangsstellung drehbar sind, aus welcher heraus das distale Schalenteil (2) relativ zum proximalen Schalenteil (3) zur Durchführung der Umwendebewegung entgegen der Gegenkraft drehbar ist.

10. Orthese (1) nach Anspruch 9, bei der das distale Schalenteil (2) aus der Ausgangsstellung heraus in beide Umwenderichtungen drehbar ist und das Gegenkraftelement (5) dazu ausgelegt ist, in beiden Umwenderichtungen eine der Drehung entgegenwirkende Gegenkraft auszuüben.

11. Orthese (1) nach einem der vorangehenden Ansprüche, mit einem Streckanschlag (3a) für das Ellenbogengelenk.

## Claims

1. An orthosis (1) for guiding a turning movement of a forearm about its biomechanical axis of rotation, comprising
a distal shell part (2) which is designed to non-rotatably grasp the forearm and/or the hand in a contact portion (2b) which is located in the hand/wrist region (d) extending from the distal ulnaradius joint up to the distal end of the metacarpus, so that the distal shell part (2) and the hand/wrist region (d) jointly are rotatable about the biomechanical axis of rotation of the forearm,
a proximal shell part (3) designed to non-rotatably grasp the elbow relative to the upper arm, and comprising
a rotary joint (4, 20) with an axis of rotation which in the applied condition of the orthosis corresponds with the biomechanical axis of rotation of the forearm and around which the distal shell part (2) is rotatable relative to the proximal shell part (3) for carrying out the turning movement,
wherein the rotary joint (4, 20) connects the two shell parts (2, 3) with each other and the connection is rigid,
**characterized by**
a counterforce element (5) attached to one of the two shell parts (2, 3), which during the execution of a turning movement of the forearm exerts a counterforce acting against the turning movement.

2. The orthosis (1) according to claim 1, in which the distal shell part (2) is designed to grasp the metacarpus.

3. The orthosis (1) according to claim 1 or 2, in which the rotary joint (4) in a plane vertical to the longitudinal axis of rotation includes an inner circular arc-shaped joint portion and an overlying outer circular arc-shaped joint portion, which for carrying out the turning movement can be guided past each other.

4. The orthosis (1) according to any of claims 1 to 3, comprising a motor for driving the rotation of the distal shell part (2).

5. The orthosis (1) according to any of the preceding claims, in which the counterforce element (5) is a spring element (5).

6. The orthosis (1) according to claim 5, in which the counterforce element (5) is a gas pressure spring (5).

7. The orthosis (1) according to claim 5 or 6, in which the spring element (5) is connected with the other one of the two shell parts (2, 3) via a Bowden cable (6, 12).

8. The orthosis (1) according to claim 7, in which the spring element (5) is aligned parallel to the axis of rotation of the rotary joint.

9. The orthosis (1) according to any of the preceding claims, in which the two shell parts (2, 3) are rotatable relative to each other around the axis of rotation of the rotary joint free from counterforce into a starting position out of which the distal shell part (2) is rotatable relative to the proximal shell part (3) for carrying out the turning movement against the counterforce.

10. The orthosis (1) according to claim 9, in which the distal shell part (2) is rotatable out of the starting position in both turning directions and the counterforce element (5) is designed to exert a counterforce acting against the rotation in both turning directions.

11. The orthosis (1) according to any of the preceding claims, comprising an extension stop (3a) for the elbow joint.

## Revendications

1. Orthèse (1) pour exécuter un mouvement de retournement d'un avant-bras autour de son axe de rotation biomécanique, comprenant
une partie distale en forme de coque (2) qui est conçue pour saisir solidairement en rotation l'avant-bras et/ou la main dans une partie de contact (2b) qui se trouve dans la région main/poignet (d) s'étendant depuis l'articulation distale cubitus/radius jusqu'à l'extrémité distale du métacarpe, de sorte que la partie distale en forme de coque (2) et la région main/poignet (d) sont capables de rotation conjointement autour de l'axe de rotation biomécanique de l'avant-bras,
une partie proximale en forme de coque (3) qui est conçue pour saisir solidairement en rotation le coude par rapport à l'avant-bras, et comprenant
une articulation rotative (4, 20) avec un axe de rotation qui, dans l'état mis en place de l'orthèse, coïncide avec l'axe de rotation biomécanique de l'avant-bras et autour duquel la partie distale en forme de coque (2) est capable de rotation par rapport à la partie proximale en forme de coque (3) pour exécuter le mouvement de retournement,
dans laquelle l'articulation rotative (4, 20) relie l'une à l'autre les deux parties en forme de coque (2, 3) et la liaison est réalisée rigide vis-à-vis de la flexion,
**caractérisée par**
un élément d'effort antagoniste (5) fixé à l'une des deux parties en forme de coque (2, 3) qui, pendant l'exécution d'un mouvement de retournement de l'avant-bras, exerce un effort antagoniste s'opposant au mouvement de retournement.

2. Orthèse (1) selon la revendication 1, dans laquelle la partie distale en forme de coque (2) est conçue pour saisir le métacarpe.

3. Orthèse (1) selon la revendication 1 ou 2, dans laquelle l'articulation rotative (4) comprend, dans un plan perpendiculaire à l'axe de rotation longitudinal, une portion d'articulation intérieure en forme d'arc de cercle et une portion d'articulation extérieure en forme d'arc de cercle situé au-dessus, qui peuvent être passées l'une à côté de l'autre pour exécuter le mouvement de retournement.

4. Orthèse (1) selon l'une des revendications 1 à 3, comprenant un moteur pour l'entraînement de la rotation de la partie distale en forme de coque (2).

5. Orthèse (1) selon l'une des revendications précédentes, dans laquelle l'élément d'effort antagoniste (5) est un élément de ressort (5).

6. Orthèse (1) selon la revendication 5, dans lequel l'élément d'effort antagoniste (5) est un ressort pneumatique à gaz (5).

7. Orthèse (1) selon la revendication 5 ou 6, dans laquelle l'élément de ressort (5) est relié à l'autre des deux parties en forme de coque (2, 3) via un système à câble (6, 12).

8. Orthèse (1) selon la revendication 7, dans laquelle l'élément de ressort (5) est orienté parallèlement à l'axe de rotation de l'articulation rotative.

9. Orthèse (1) selon l'une des revendications précédentes, dans laquelle les deux parties-en forme de coque (2, 3) sont capables de tourner l'une par rapport à l'autre autour de l'axe de rotation de l'articulation rotative en l'absence d'un effort antagoniste jusque dans une position de départ, à partir de laquelle la partie distale en forme de coque (2) est capable de tourner par rapport à la partie proximale en forme de coque (3) pour exécuter le mouvement de retournement à l'encontre de l'effort antagoniste.

10. Orthèse (1) selon la revendication 9, dans laquelle la partie distale en forme de coque (2) est capable de tourner, à partir de la position de départ, dans les deux directions de retournement, et l'élément d'effort antagoniste (5) est conçu pour exercer dans les deux directions de retournement un effort antagoniste s'opposant à la rotation.

11. Orthèse (1) selon l'une des revendications précédentes, comprenant une butée de limitation de course (3a) pour l'articulation du coude.
